# EUROPÄISCHE PATENTSCHRIFT

(11) **EP 2 616 125 B1**
(45) Veröffentlichungstag und Bekanntmachung des Hinweises auf die Patenterteilung: **17.05.2017**
(21) Anmeldenummer: 11754296.9
(22) Anmeldetag: 30.08.2011
(51) Int. Cl.: A61M 5/34

(54) **SPRITZENKÖRPER-KANÜLEN-VERBUND UND VERFAHREN ZU DESSEN HERSTELLUNG**
SYRINGE BODY/NEEDLE ASSEMBLY AND METHOD FOR PRODUCING SAME
ASSEMBLAGE CORPS DE SERINGUE-CANULE ET SON PROCÉDÉ DE FABRICATION

(30) Priorität: 13.09.2010 DE 102010045095
(43) Veröffentlichungstag der Anmeldung: 24.07.2013
(73) Patentinhaber: Schott AG, 55122 Mainz (DE)
(72) Erfinder: HOPPE, Bernd, 55218 Ingelheim (DE); SEILER, Daniela, 55232 Alzey (DE); ROTERS, Andreas, 55118 Mainz (DE); SCHELLENBERG, Kathrin, 65239 Hochheim (DE)
(74) Vertreter: Blumbach Zinngrebe
(86) Internationale Anmeldenummer: PCT/EP2011/004364
(87) Internationale Veröffentlichungsnummer: WO 2012/034648

(56) Entgegenhaltungen:
- EP-A1- 2 025 357
- WO-A1-02/066387
- WO-A1-2006/136364
- US-A1- 2002 138 042

## Beschreibung

Die Erfindung betrifft einen Spritzenkörper-Kanülen-Verbund und ein Verfahren zur Herstellung eines solchen Spritzenkörper-Kanülen-Verbunds. Insbesondere betrifft die Erfindung ein Verfahren, mit welchem auf die Verwendung von organischem Kleber zur Verbindung von Spritzenkörper und Kanüle verzichtet werden kann.

Für die Herstellung von Spritzen, z.B. für Pharmaapplikationen, wird üblicherweise durch mehrstufige Heißumformungsprozesse aus einem Glasröhrchen der eigentliche Spritzenkörper geformt. Der geformte Spritzenkörper wird dann inspiziert und anschließend in einem Kühlofen gekühlt.

Bei Spritzensystemen, die mit dem zu applizierende Medikament befüllt ausgeliefert werden, ist häufig auch schon die Kanüle integriert. Dazu kann nach der Herstellung des Spritzenkörpers die Kanüle in die Düse (auch als Spritzenkonus bezeichnet) der Spritze eingeklebt werden. Der Spritzenkonus kann dabei einen beliebigen, nicht zwangsweise konvergenten, Verlauf haben. Unter einem Spritzenkonus werden im Folgenden alle Düsen mit Querschnittsverläufen verstanden, deren Aufgabe in der Verringerung des fluidführenden Querschnitts gegenüber dem Kolbendurchmesser besteht.

Die Kanüle wird dann zunächst in die vorgesehene Position in der Düse eingebracht und anschließend mittels eines flüssigen organischen Klebers, der bei Raumtemperatur zugegeben und beispielsweise unter ultraviolettem Licht ausgehärtet wird, fixiert. Danach wird der so hergestellte Spritzenkörper-Kanülen-Verbund gereinigt, die Innenwand des Spritzenkörpers silikonisiert und ein Verschluss auf Kanüle und Düse aufgesetzt. Anschließend wird dieses Spritzensystem im Verbund sterilisiert.

Die Befüllung der Spritze erfolgt dann bei den pharmazeutischen Unternehmen, die auch den abschließenden Stopfen mit dem Stempel beispielsweise zur Injektion des Medikaments setzen und die Einzelverpackung vornehmen.

Während der Lagerungszeit der vorgefüllten Spritze steht das Medikament permanent mit dem organischen Kleber, der die Kanüle in der Spritzendüse fixiert, in Kontakt. Durch den direkten Kontakt zwischen Medikament und Kleber kann es zur unerwünschten Eindiffusion von Kleberbestandteilen in das Medikament und auch zu Interaktionen zwischen Medikament und Kleberbestandteilen kommen. Diese Effekte können durch ungenügend ausgehärtete Kleber noch verstärkt werden.

Darüber hinaus kann beim Einkleben der Kanüle auch Kleber in die Kanüle gelangen. Dies ist insbesondere dann problematisch, wenn beispielsweise ein UV-härtbarer Kleber verwendet wird. Der in die Kanüle gelangte Kleber wird durch die metallische Kanüle vom eingestrahlten UV-Licht abgeschirmt und härtet daher nicht oder jedenfalls nur ungenügend aus. Nicht ausgehärtete Kleberbestandteile können dann mit dem eingebrachten Medikament interagieren. Insbesondere Proteine oder auch DNA- und/oder RNA-Komplexe können sehr empfindlich auf geringste Verunreinigungen reagieren. Solche Verunreinigungen können Konformationsänderungen bewirken, die dann die Wirkung der Medikamente stark verringern oder sogar ganz aufheben können. Da in der modernen Pharmazie immer mehr solcher empfindlichen Medikamente entwickelt, produziert und appliziert werden, wird die Verringerung von möglichen Kontaminationsquellen immer wichtiger.

Darüber hinaus sind sowohl der Silikonisierungsprozess als auch die Sterilisationsprozesse meist mit erhöhten Temperaturen verbunden. Da diese Prozesse nach dem Einkleben der Kanüle in den Spritzenkörper durchgeführt werden, limitiert die Temperaturbeständigkeit des Klebers die möglichen Methoden, die für diese Prozesse verwendet werden können. Handelsübliche Kleber sind heute bis maximal 150°C temperaturbeständig. Um jedoch 100% pyrogenfreie Verpackungsmaterialien bereitstellen zu können, ist eine Einbrenn-Silikonisierung bei über 300°C erforderlich. Viele handelsübliche UV-härtende Kleber verlieren jedoch bei diesen Temperaturen ihre Festigkeit.

Die Fixierung von Kanülen in pharmazeutischen Spritzen mittels eines organischen Klebers wird beispielsweise in den Dokumenten US 3,194784 und US 3,390,678 beschrieben.

Neben der Verwendung von organischen Klebern sind jedoch auch andere Verfahren zum Befestigen der Kanüle im oder am Spritzenkörper bekannt. So beschreibt die Patentschrift US 3,364,002 ein Verfahren, in dem eine Kanülenhalterung direkt in aufgeschmolzenes Glas einer Spritze eingebracht und so ein Schmelzverbund hergestellt wird. Dabei wird jedoch die Kanülenhalterung auf sehr hohe Temperaturen aufgeheizt, was zu einer Schädigung des Metalls, z.B. durch Korrosion, führen kann.

Aus der US 2002/138042 A1 ist eine Zylinderanordnung für eine Injektionsspritze bekannt, die einen länglichen Zylinder umfasst mit einem distalen Ende, das eine Spitze zum Halten einer Nadelkanüle aufweist. Die Nadelkanüle ist an der Spitze mit einem Bindemittel befestigt, welches getrocknet und/oder gehärtet werden kann unter Verwendung von Wärme, sichtbarem Licht, Infrarot Licht und/oder UV-Licht.

Aus der WO 02/066387 A1 ist eine Vorrichtung zur Fertigung einer Spritze mit einen Spritzenkörper aus Glas und einer Kanüle. Hierzu ist eine Vorrichtung vorgesehen, die eine Fügezone zwischen Spritzenkörper und Kanüle mit Laserstrahlung beaufschlagt, um das Material des Spritzenkörpers anzuschmelzen, damit sich dieses während der Materialverfestigung dauerhaft mit der Kanüle verbindet.

Aus der EP 2 025 357 A1 ist ein Spritzenkörper mit einen zylindrischen Abschnitt bekannt, wobei in einem Aufnahmekanal eines axialen Endbereichs des zylindrischen Abschnitts eine Kanüle angeordnet ist. Die Kanüle ist im Aufnahmekanal an mindestens einem Befestigungsbereich durch Material des zylindrischen Abschnitts festgelegt. Mittels eines Lasers wird der Befestigungsbereich aufgeschmolzen, so dass sich die Schmelze des Glases in den Bereichen um den Umfang der Kanüle legt und eine Verbindung zwischen zylindrischem Abschnitt und Kanüle bildet.

Aus der WO 2006/136364 A1 ist eine Kanüle für eine Injektionsvorrichtung bekannt, mit einen festen und einen biegsamen Anschnitt. Der feste Abschnitt umfasst eine Spitze, die geeignet ist, in die Haut eines Patienten einzudringen. Beim Herstellen der Kanüle wird ein Teil der Kanüle mit Ausnahme des Spitzenteils Wärme oder einem magnetischen Wechselfeld ausgesetzt, um den biegsamen Abschnitt auszubilden.

Der Erfindung liegt daher die Aufgabe zugrunde, ein kostengünstiges Verfahren zur Verfügung zu stellen, mithilfe dessen ein Spritzenkörper-Kanülen-Verbund hergestellt werden kann, der auf eine Verklebung mittels organischem Kleber verzichtet.

Eine weitere Aufgabe der Erfindung besteht in der Bereitstellung eines Verfahrens zur Herstellung eines Spritzenkörper-Kanülen-Verbunds, das ausschließlich anorganische Materialien in Bereichen, die mit eingefüllten Medikamenten in Berührung kommen, verwendet.

Weiterhin ist es Aufgabe der Erfindung, einen Spritzenkörper-Kanülen-Verbund bereitzustellen, der den oben genannten Aufgaben gerecht wird.

Die Aufgabe der Erfindung wird durch den Gegenstand der unabhängigen Ansprüche gelöst. Vorteilhafte Weiterbildungen der Erfindung sind in den jeweiligen abhängigen Ansprüchen angegeben.

Demgemäß umfasst ein erfindungsgemäßes Verfahren zur Herstellung eines Spritzenkörper-Kanülen-Verbunds zumindest die folgenden Schritte:
- Bereitstellen einer Kanüle (20) und eines Spritzenkörpers (10) mit Düse (11) und Düsenkanal, wobei ein erstes Material, das im Bereich der Düse (11) bereitgestellt wird, und zumindest einen Verbindungskörper (30) umfasst, mehr elektromagnetische Strahlung (41) absorbiert als ein zweites Material des restlichen Spritzenkörpers (10);
- Anordnen zumindest des Spritzenkörpers (10) und der Kanüle (20) derart, dass die Kanüle (20) mit dem Spritzenkörper (10) verbunden werden kann und im verbundenen Zustand eine hermetisch dichte Verbindung zwischen dem Spritzenkörper (10) und der Kanüle (20) vorliegt;
- Herstellen der Verbindung zwischen Spritzenkörper (10) und Kanüle (20), wobei das erste Material im Bereich der Düse (11) zumindest lokal mit elektromagnetischer Strahlung (41) bestrahlt wird und dabei zumindest bereichsweise aufschmilzt und die Kanüle (20), hermetisch dichtend, im Spritzenkörper (10) fixiert;
- Abkühlen des Spritzenkörper-Kanülen-Verbunds (1).

Gemäß der Erfindung absorbiert das erste Material im Bereich der Düse mehr elektromagnetische Energie als das zweite Material des restlichen Spritzenkörpers. Beispielsweise ist die totale Energieabsorption des ersten Materials im Bereich der Düse im Wellenlängenbereich der eingesetzten Strahlungsquelle beim Einfachdurchgang der Strahlung zumindest absolut 10% höher, bevorzugt zumindest absolut 30% höher und insbesondere bevorzugt zumindest absolut 50% höher als die des zweiten Materials des restlichen Spritzenkörpers. Die Düse des Spritzenkörpers kann typischerweise konusförmig sein; jedoch sind auch andere Formen grundsätzlich umfasst.

Im Unterschied zu konventionellen Verfahren oder Verfahren, bei welchen die Erwärmung des Materials durch Strahlungsabsorption im Bereich der Materialoberfläche geschieht, kennzeichnet die Erfindung eine aufgrund der vorzugsweise im Kanal der Düse, beziehungsweise des Konus angeordneten, stärker absorbierenden ersten Materials von innen nach außen erfolgende Erwärmung. Die Innenseite des Kanals wird also schneller erwärmt, als die Außenseite der Düse, obwohl die Strahlung durch das Material des Spritzenkörpers hindurchtritt. Es ergibt sich mit anderen Worten somit ein Temperaturgradient von innen nach außen.

Gemäß einer Ausführungsform der Erfindung kann die erhöhte Strahlungsabsorption durch eine chemische oder strukturelle Modifikation der Oberfläche beispielsweise der Düse erzielt werden. Beispielsweise kann durch eine geeignete Oberflächenbehandlung z.B. Ionenaustausch, Ionen in die Oberfläche des Innenkanals der Düse diffundieren, so dass eine lokale Erhöhung der Strahlungsabsorption im Bereich der eindiffundierten Ionen erfolgt. Analog kann eine Oberflächenmodifikation durch ein Aufrauen beispielsweise durch Sandstrahlen oder Ätzen erzielt werden. Auch ist eine Beschichtung mit einem geeigneten, höher strahlungsabsorbierenden Material denkbar. Auf diese Weisen kann auch die Innenfläche der Düse des Spritzenkörpers behandelt werden und so ganz auf zusätzliches Verbindungsmaterial verzichtet werden.

Darüber hinaus liegt im Rahmen der Erfindung auch ein Spritzenkörper mit Kanüle, umfassend zumindest einen Spritzenkörper mit Düse, welche eine Öffnung aufweist, und eine Kanüle. Dieser Spritzenkörper mit Kanüle ist mit dem oben beschriebenen Verfahren herstellbar oder hergestellt. Die Kanüle ist im Spritzenkörper fixiert und die Verbindung der Öffnung durch die die Kanüle mit dem Inneren des Spritzenkörpers verbunden ist, ist hermetisch zur Kanüle abgedichtet. Dabei umfasst der Bereich der Düse ein erstes Material, das mehr elektromagnetische Strahlung absorbiert als das zweite Material aus dem der restliche Spritzenkörper gefertigt ist. Die Kanüle ist in das erste Material, das mehr elektromagnetische Strahlung absorbiert, eingeschmolzen. Beispielsweise ist die totale Energieabsorption des ersten Materials im Bereich der Düse im Wellenlängenbereich der eingesetzten Strahlungsquelle beim Einfachdurchgang der Strahlung zumindest absolut 10% höher, bevorzugt zumindest absolut 30% höher und insbesondere bevorzugt zumindest absolut 50% höher als die des zweiten Materials des restlichen Spritzenkörpers.

Das erste Material im Bereich der Düse umfasst einen Verbindungskörper, der in der Düse angeordnet ist. Dabei umfasst der Verbindungskörper ein Material, das mehr elektromagnetische Strahlung absorbiert als das zweite Material des Spritzenkörpers.

Um unnötige Wiederholungen zu vermeiden, beziehen sich die beschriebenen Merkmale sowohl auf das Verfahren als auch auf das Produkt, soweit dies sinnvoll ist und nichts anderes angemerkt ist.

Das erfindungsgemäße Verfahren ist kostengünstig und ermöglicht das Verbinden einer Kanüle mit einem Spritzenkörper, vorzugsweise unter der ausschließlichen Verwendung von anorganischem Material, insbesondere mittels Glas. Gemäß einer bevorzugten Ausführungsform der Erfindung verhindert dieses Verfahren darüber hinaus auch den Aufbau von Restspannungen in einem beispielsweise glasigen Spritzenkörper.

Die Erfindung ist aber ebenso auf Kunststoffe als Material des Spritzenkörpers und/oder des Verbindungskörpers anwendbar. Ein besonders bevorzugtes Material für Kunststoffspritzen ist dabei COC (Cyclo-Olefin-Copolymer). Gemäß einer ersten Variante der Ausführungsform der Erfindung unter Verwendung von Kunststoff, vorzugsweise COC wird Kunststoff sowohl für den Spritzenkörper, als auch den Verbindungskörper eingesetzt. Gemäß einer zweiten Variante wird ein Glas-Spritzenkörper verwendet, wobei aber ein Kunststoff-Verbindungskörper eingesetzt wird, um die Kanüle mit dem Spritzenkörper zu verbinden. Gegebenenfalls ist keine Integration strahlungsabsorbierender Stoffe in den Verbindungskörper erforderlich, wenn in der ersten Variante verschiedene Kunststoffe mit unterschiedlichem spektralen Absorptionsverlauf verwendet werden, oder sich in der zweiten Variante die spektralen Absorptionsverläufe von Glas und Kunststoff hinreichend unterscheiden und ein entsprechender Spektralbereich für die eingestrahlte Strahlung ausgewählt wird, bei welchem die Absorption im Verbindungskörper höher ist als im Material des Spritzenkörpers. Bei den Varianten der allgemeineren Ausführungsform der Erfindung unter Verwendung von Kunststoffen gemeinsam ist, dass ein Kunststoff-Verbindungskörper verwendet wird, wobei der Spritzenkörper sowohl aus Glas, als auch aus Kunststoff gefertigt sein kann.

Der Spritzenkörper kann vorzugsweise eine integrale, d.h. aus dem Spritzenkörper ausgeformte Düse aufweisen, wobei es sich beispielsweise um eine randständige Verengung des Spritzenkörpers handeln kann. In oder an dieser Düse kann gemäß einer besonders bevorzugten Ausführungsform der Erfindung der Verbindungskörper ein- oder angeschmolzen werden, der die Kanüle oder Nadel der Spritze am Spritzenkörper fixiert.

Gemäß einer weiteren vorteilhaften Ausführungsform der Erfindung wird als Material für den Spritzenkörper und/oder den zumindest einen Verbindungskörper Glas verwendet. Gerade bei der Verwendung eines Spritzenkörpers und eines Verbindungskörpers aus Glas kann dann auf organische Bestandteile zum Verbinden der Nadel in der Spritze ganz verzichtet werden.

Dabei kann der zumindest eine Verbindungskörper einen Glaskapillarabschnitt und/oder einen Sinterkörper aus Glas und/oder Glaspulver und/oder Glaspaste umfassen. Unter Glaspaste wird dabei mit Flüssigkeit gebundenes Glaspulver verstanden.

Insbesondere unterscheidet sich das erste Material des Verbindungskörpers chemisch und/oder strukturell vom dem zweiten Material des Spritzenkörpers.

Vorteilhaft kann der Verbindungskörper beispielsweise dotiertes Glas, insbesondere mit einem die zur Erwärmung verwendete Strahlung absorbierenden Bestandteil dotiertes Glas umfassen. Auf diese Weise ist es z.B. möglich, Glas nahezu gleicher Zusammensetzung für den Spritzenkörper und den Verbindungskörper zu verwenden und trotzdem sicherzustellen, dass das erste Material des Verbindungskörpers mehr elektromagnetische Energie absorbiert als das zweite Material des Spritzenkörpers. Insbesondere vorteilhaft kann der Verbindungskörper mit Verbindungen von Chrom, Nickel, Kupfer, Eisen, Kobalt, seltene Erden (z.B. Ytterbium, Dysprosium) oder andere im interessierende Wellenlängenbereich absorbierenden Elementen, Materialien oder Verbindungen dotiert sein. Auch Kombinationen der vorgenannten Verbindungen sind möglich. Es ist jedoch auch möglich, einen Sinterkörper aus Glas als Verbindungskörper zu verwenden, wobei dem Glasausgangspulver ein Material zugemischt wird, dass die Absorption elektromagnetischer Strahlung erhöht. Beispielsweise kann das Glaspulver für einen solchen Sinterkörper mit einem absorbierenden Pigment gemischt sein. Auf diese Art können auch absorbierende Verbindungen eingebracht werden, die ansonsten im Glasschmelzprozess für das Ausgangspulver des Sinterkörpers signifikanten Änderungen unterliegen würden und wesentliche Eigenschaften verlieren würden. Auch ist es möglich, dass oben genannte Glasdotierungsstoffe in diesem Prozessschritt eingebracht werden. Von Vorteil ist hierbei, dass als Ausgangspulver für die Verbindungskörper das gleiche Material wie für den restlichen Spritzenkörper Verwendung finden kann.

Gemäß einer bevorzugten Ausführungsform der Erfindung wird ein vorzugsweise ringförmiger anorganischer Verbindungskörper oder Bondkörper zwischen die Kanüle und die Düsenwand eines Spritzenkörpers eingebracht. Dieser Verbindungskörper kann durch verstärkte Absorption von energiereicher elektromagnetischer Strahlung lokal aufgeheizt beziehungsweise aufgeschmolzen werden, so dass die Kanüle mit der Düse hermetisch verbunden wird. Selbstverständlich bezieht sich die hermetische Verbindung zwischen Kanüle und Spritzenkörper nicht auf die Kanüle selber, die ja weiterhin funktionsfähig sein soll. Das heißt, dass die Kanüle trotz der hermetischen Verbindung zwischen Kanüle und Spritzenkörper natürlich zum Injizieren des später in die Spritze einzufüllenden Medikaments geeignet ist.

Unter elektromagnetische Strahlung im Sinne der Erfindung wird jede elektromagnetische Strahlung verstanden, die eine Erwärmung bis zur notwendigen Verbindungstemperatur des ersten Materials im Bereich der Düse, beispielsweise eines Verbindungskörpers, bewirkt. Insbesondere bevorzugt handelt es sich jedoch um elektromagnetische Strahlung, die nicht oder nur sehr wenig vom zweiten Material des Spritzenkörpers, jedoch gut oder sehr gut vom ersten Material im Bereich der Düse absorbiert wird.

Besonders bevorzugt erfolgt die Bestrahlung mittels elektromagnetischer Strahlung durch einer oder mehreren UV-Strahlungsquellen und/oder Strahlungsquellen, die im sichtbaren Bereich abstrahlen und/oder Infrarot-Strahlungsquellen, insbesondere Infrarot-Strahlungsquellen, die kurzwellige Infrarotstrahlung abgeben, und/oder Mikrowellenstrahlungsquellen.

Als UV-Strahlungsquelle können beispielsweise Quecksilberdampflampen eingesetzt werden. Als Strahlungsquelle, die im sichtbaren Bereich abstrahlt, können Xenon-Kurzbogen-Hochdrucklampen verwendet werden. Als Infrarot-Strahlungsquellen sind Laser, beispielsweise Nd:YAG-Laser, oder Temperaturstrahler, beispielsweise Wolfram-IR-Strahler, geeignet. MikrowellenStrahlungsquellen, die im Rahmen der Erfindung eingesetzt werden können umfassen beispielsweise Magnetrons.

Besonders bevorzugt wird beispielsweise für ungefärbte Neutralgläser kurzwellige Infrarotstrahlung (kIR-Strahlung), insbesondere im Bereich um etwa 1 µm, verwendet. Bevorzugte Wellenlängenbereiche der verwendeten kIR-Strahlung reichen von 800 bis 3000 Nanometer, besonders bevorzugt von 800 bis 1500 Nanometer. Eine solche elektromagnetische Strahlung kann beispielsweise mittels eines Wolfram-Halogen-IR-Strahlers, der eine Farbtemperatur von 1500 K - 3500 K aufweisen kann, erzeugt werden.

Bei der Verwendung einer solchen Heiztechnologie wird die Erwärmung nicht allein durch die Temperatur des Ofens, sondern wesentlich durch die IR-Strahlung der Heizelemente und das Absorptionsverhalten des aufzuheizenden Körpers bestimmt. Durch die gezielte Auswahl und Einstellung der Strahlungs- und Absorptionsverhältnisse kann so Glas, beispielsweise ein hoch-kurzwellig IRstrahlungsabsorbierender Verbindungskörper in der Düse des niedrig-strahlungsabsorbierenden Spritzenkörpers, gezielt lokal erhitzt und zumindest teilweise aufgeschmolzen werden.

Unterschiede in der resultierenden Strahlungsabsorption zwischen Verbindungskörper und Spritzenkörper können u.a. durch die Verwendung verschiedener Glasarten, durch Dotierung mit färbenden Elementen oder durch Strukturunterschiede, z.B. durch das Einbringen von Streuzentren oder die Verwendung von Sinterkörpern, erreicht werden.

Besonders bevorzugt wird beim Schritt des Herstellens der Verbindung zwischen Spritzenkörper und Kanüle der zumindest eine Verbindungskörper zumindest teilweise aufgeschmolzen. Das aufgeschmolzene erste Material des Verbindungskörpers liegt dabei nach dem Abkühlen an der Wandung des Spritzenkörpers und um die Kanüle derart an, dass die Kanüle hermetisch dicht in den Spritzenkörper eingefügt ist.

Bei der Verwendung von Verbindungskörpern können gemäß einer bevorzugten Ausführungsform sowohl Glasrohrabschnitte als auch gesinterte Glasröhrchen zum Einsatz kommen. Darüber hinaus ist jedoch auch eine Verbindung zwischen Kanüle und Spritzenkörper durch lokales Aufschmelzen von Glaspulver oder Glaspaste möglich.

Der Verbindungskörper, der Spritzenkörper und Kanüle verbindet, ist gemäß einer Ausführungsform der Erfindung in oder an der Düse des Spritzenkörpers angeordnet. Bevorzugte Ausführungsformen der Erfindung sehen vor, den Verbindungskörper am oberen oder am unteren Ende der Düse anzuordnen. Es ist jedoch auch eine mittige Anordnung möglich. Die Düse kann durch den Verbindungskörper ganz oder teilweise ausgefüllt sein. Darüber hinaus kann der Verbindungskörper auch auf das proximale oder distale Ende der Düse aufgesetzt sein. Anders ausgedrückt bedeutet dies, dass der Verbindungskörper in der proximalen Anordnung auf die Düse vom Inneren des Spritzenkörpers her aufgesetzt ist. In der distalen Anordnung ist der Spritzenkörper am freien Düsenende, also dem eigentlichen Spritzenkörper gegenüber, angeordnet.

Gemäß einer weiteren bevorzugten Ausführungsform der Erfindung wird nicht nur ein Verbindungskörper zum Befestigen der Kanüle im Spritzenkörper verwendet, sondern mehrere. So kann die Kanüle beispielsweise durch zwei Verbindungskörper, z.B. im oberen und unteren Bereich der Düse, in die Düse eingeschmolzen werden.

Vorteilhaft kann die Düse des Spritzenkörpers auch Aussparungen aufweisen, in die der Verbindungskörper passgenau eingefügt werden kann. Dies erleichtert zum Einen die Anordnung von Spritzenkörper, Verbindungskörper und Kanüle vor dem eigentlichen Verbinden dieser Komponenten. Andererseits kann es auch die Verbindung im fertigen Spritzenkörper-Kanülen-Verbund stabilisieren, indem die Aussparung als Widerlager gegen den beim Spritzen ausgeübten Druck auf den Verbindungskörper wirkt. Dies ist dann der Fall, wenn die Aussparung sich in proximale Richtung, also zum Spritzenkörper hin, öffnet. Es ist natürlich auch möglich, eine Aussparung vorzusehen, die sich in distale Richtung, also zum freien Düsenende hin, öffnet.

Neben dem lokalen Eintrag der elektromagnetischen Energie insbesondere in den Verbundkörper, der Spritzenkörper und Kanüle verbinden soll, kann auch zumindest der Spritzenkörper erwärmt werden. Insbesondere kann der Spritzenkörper auf eine Temperatur erwärmt werden, bei dem sein Material, beziehungsweise das zweite Material zumindest den unteren Kühlpunkt erreicht. Bei der Verwendung von Glas liegt der untere Kühlpunkt bei einer Viskosität η von 10^{14,5} d·Pas. Bei einer solchen Viskosität ist das Glas des Spritzenkörpers noch formstabil, aber trotzdem in der Lage Spannungen zu relaxieren, die durch die Verwendung von Materialien mit unterschiedlichen thermischen Ausdehnungskoeffizienten entstehen können.

Dabei kann das zusätzliche Erwärmen zumindest des Spritzenkörpers beispielsweise mittels Infrarotstrahlung und/oder mittels eines Heißluftstroms erfolgen. Grundsätzlich sind viele konventionelle Heizvorrichtungen hier anwendbar. Besonders bevorzugt wird der Spritzenkörper gleichzeitig mit dem Erwärmen des Verbindungskörpers durch die gleiche Strahlungsquelle aufgeheizt. Dabei ist beispielsweise der Unterschied in der Absorption von Spritzenkörper und Verbindungskörper so gewählt, dass der Verbindungskörper die Verbindungstemperatur und der Spritzenkörper den unteren Kühlpunkt erreicht.

Besonders bevorzugt kann sich an den Schritt des Herstellens der Verbindung zwischen Spritzenkörper und Kanüle noch eine thermische Nachbehandlung anschließen. Diese thermische Nachbehandlung, bei der der Spritzenkörper-Kanülen-Verbund nochmals, insbesondere auf zumindest die Temperatur des Unteren Kühlpunkts, erhitzt wird, relaxiert vorteilhaft noch vorhandene, durch unterschiedliche thermische Ausdehnungskoeffizienten der Materialien und/oder unterschiedlich stark erwärmte Bereiche des Spritzenkörpers und der Kanüle induzierte Restspannungen in zumindest dem Spritzenkörper.

Beim Einsatz eines nach dem Stand der Technik eingesetzten CO₂-Lasers als Strahlungsquelle, der naturgemäß im Wellenlängenbereich von 10 µm emittiert, wird die Strahlung direkt an der Oberfläche des Spritzenkörpers absorbiert und nicht bevorzugt der Verbindungskörper erwärmt. Weitere Nachteile wie beispielsweise komplizierte und aufwendige Strahlführung, Risiko der schnellen Überhitzung der Oberfläche der Spritzendüse, überhöhte Restspannungen im verbundenen Spritzenkörper und zeitintensive Prozesse durch Einzelstückbearbeitung werden mit der hier vorgestellten Technologie vermieden.

Darüber hinaus kann eine Überhitzung der Stahlkanüle durch die gezielte lokale Aufheizung des Verbindungskörpers vermieden werden. Durch die Einstellung der Strahlungsabsorption des Verbindungskörpers ist es außerdem möglich, die Prozesszeit so zu optimieren, dass bei einer etwaigen kritischen Temperaturerhöhung der einzubindenden Metallkanüle keine unzulässige Schädigung dieser auftritt. Weiterhin kann die Korrosion des Kanülenmetalls, insbesondere durch die zusätzliche Verwendung von Stickstoff oder anderen Inertgasen im Bereich der Kanüle, vermindert beziehungsweise gänzlich vermieden werden.

Überraschenderweise wurde bei den durchgeführten Verbindungsversuchen festgestellt, dass keine Spannungsrisse insbesondere im verbundenen Bereich, aber auch nicht im sonstigen Spritzenkörper auftreten. Dies war anfänglich nicht als möglich eingestuft worden, da die Ausdehnungskoeffizienten der Metallkanüle (10 - 20 x 10⁻⁶/K) und des Glases des Verbindungskörpers beziehungsweise des Spritzenkörpers (0 - 5 x 10⁻⁶/K) sehr weit auseinander liegen. Es wurde davon ausgegangen, dass unweigerlich Spannungen auftreten müssten. Messungen an verbundenen Spritzenkörpern haben jedoch gezeigt, dass die verbleibenden Restspannungen weit unter den zulässigen kritischen Rissspannungen für den Spritzenkörper liegen. Es ließ sich durch die Versuche nachweisen, dass die starken Unterschiede in den Ausdehnungskoeffizienten von Glas und Metall nicht notwendigerweise zu überhöhten Restspannungen führen müssen, da über die lokale Temperaturführung in geeigneter Art und Weise geringe Restspannungen eingestellt werden können. Unterschiede im Ausdehnungskoeffizienten führen insbesondere dann zu Restspannungen, wenn die Temperatur lokal homogen ist. Wenn, wie im hier beschriebenen Fall, die Temperatur lokal inhomogen ist, können sich die temperaturbedingten Längenänderungen angleichen und damit zu verminderten Restspannungen führen.

Bei der Herstellung der Spritzenkörper aus Glas werden zur Formung des engen Düsenkanals in der Regel Wolfram-haltige Stifte, insbesondere Stifte aus Wolfram-Metall verwendet. Dabei kann es im Heißformungsschritt zu Ablagerungen von Wolframspuren im Düsenkanal kommen. Bei empfindlichen Medikamenten kann Wolfram zu unerwünschten Wechselwirkungen mit den Wirkstoffen führen, die die Wirkung des Medikaments herabsetzen. Wird dagegen ein erfindungsgemäßer Verbindungskörper eingesetzt, kann der Düsenkanal in der Regel verbreitert sein. Bei solch vergleichsweise großen Düsenkanälen ist der Einsatz von Wolframmetallen in Form von Formwerkzeugen nicht mehr unbedingt notwendig, so dass diese unerwünschten Wechselwirkungen vermieden werden können.

### DETAILLIERTE BESCHREIBUNG DER ERFINDUNG

Im Folgenden wird die Erfindung anhand von Beispielen und Zeichnungen näher erläutert. Ähnliche oder gleiche Merkmale sind in den verschiedenen Zeichnungen mit gleichen Bezugszeichen versehen. Es zeigen:
- Fig. 1:: schematischer Querschnitt durch einen Spritzenkörper-Kanülen-Verbund zur Erläuterung des Grundprinzips der Erfindung;
- Fig. 2:: distale Anordnung des Verbindungskörpers in distal orientierter Aussparung in der Düse, wobei der Verbindungskörper nur Teile der Düse ausfüllt;
- Fig. 3:: Anordnung des Verbindungskörpers in distal orientierter Aussparung in der Düse;
- Fig. 4:: Anordnung des Verbindungskörpers ohne Aussparung in der Düse;
- Fig. 5:: mittige Anordnung des Verbindungskörpers in der Düse ohne Aussparung;
- Fig. 6:: proximale Anordnung des Verbindungskörpers in proximal orientierter Aussparung in der Düse;
- Fig. 7:: distal auf die Düse aufgesetzter Verbindungskörper;
- Fig. 8:: proximal auf die Düse aufgesetzter Verbindungskörper;
- Fig. 9:: schematischer Querschnitt durch einen Spritzenkörper-Kanülen-Verbund ohne Verbundkörper,
- Fig. 10:: schematischer Querschnitt durch einen Spritzenkörper-Kanülen-Verbund mit konischem Verbundkörper,
- Fig. 11:: schematischer Querschnitt durch einen Spritzenkörper-Kanülen-Verbund mit konischem Verbundkörper in anderer Orientierung als in Fig. 10,
- Fig. 12:: schematischer Querschnitt durch einen Spritzenkörper-Kanülen-Verbund mit Verbundkörper, der aus zwei verschiedenen Gläsern besteht.

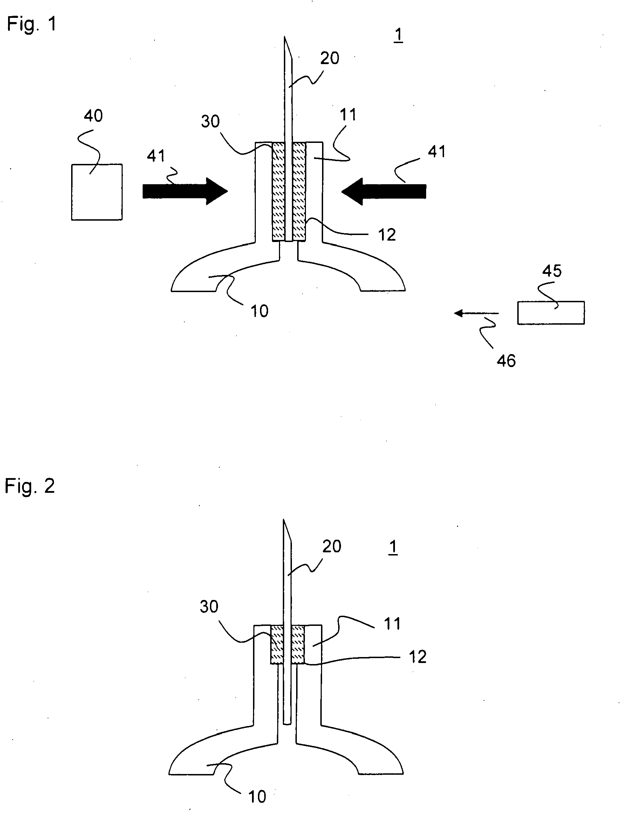

In Fig. 1 ist der grundsätzliche Aufbau eines integrierten Spritzenkörper-Kanülen-Verbunds 1 dargestellt. Der Spritzenkörper-Kanülen-Verbund 1 umfasst einen Spritzenkörper 10, eine Kanüle 20 und einen Verbindungskörper 30. Der Spritzenkörper 10 weist eine Düse 11 auf, in deren Aussparung 12 der Verbindungskörper 30 eingepasst ist. Der Verbindungskörper 30 enthält ein erstes Material, das mehr elektromagnetische Strahlung 41 absorbiert als der Spritzenkörper 10, wobei der Spritzenkörper 10 auch die Düse 11 umfasst.

Zur Herstellung des Spritzenkörper-Kanülen-Verbunds 1 werden der Spritzenkörper 10, die Kanüle 20 sowie der Verbindungskörper 30 in der späteren Verbundanordnung zueinander angeordnet. Anschließend wird der Bereich des Verbundkörpers 30 zumindest teilflächig mittels einer Vorrichtung zur Erzeugung von elektromagnetischer Strahlung 40 mit elektromagnetischer Strahlung 41 bestrahlt. Um eine möglichst selektive Erhitzung des Verbundkörpers 30 zu erreichen, besteht der Verbundkörper 30 im Wesentlichen aus einem Material, das die abgestrahlte elektromagnetische Strahlung 41 gut absorbiert. Im Gegensatz dazu bestehen der Spritzenkörper 10 und die dazugehörige Düse 11 aus einem zweiten Material, das die abgestrahlte elektromagnetische Strahlung 41 möglichst wenig absorbiert. Die elektromagnetische Strahlung 41 durchdringt das zweite Material des Spritzenkörpers 10 und der Düse 11 vorteilhaft möglichst ohne oder mit geringem Transmissionsverlust. Auf diese Weise kann eine selektive Erhitzung des Verbindungskörpers 30 erreicht werden, so dass dieser zumindest bereichsweise aufschmilzt. Das zweite Material des Spritzenkörpers 10 wird dagegen durch diese Bestrahlung 41 vorzugsweise möglichst wenig erwärmt. Dies verhindert unter anderem, dass es beim "Einschmelzen" des Verbindungskörpers 30 zu einer Deformation des Spritzenkörpers 10 kommt. Es ergibt sich somit ein vom Inneren nach außen zur Oberfläche der Düse hin abfallender Temperaturgradient.

Durch das zumindest teilweise Aufschmelzen des ersten Materials des Verbindungskörpers 11 wird zum einen die Kanüle 20 im oder an der Düse 11 fixiert. Andererseits wird jedoch auch der Spalt oder die Öffnung zwischen Düsenwand 11 und Kanüle 20 hermetisch dicht verschlossen, indem sich das aufgeschmolzene erste Material des Verbindungskörpers 30 an die Düsenwand 11 und um die Kanüle 20 herum anlegt und sich mit diesen verbindet.

Bevorzugt kann als Material für den Spritzenkörper 10 und/oder den zumindest einen Verbindungskörper 30 Glas verwendet werden. Weiterhin bevorzugt kann der zumindest eine Verbindungskörper 30 einen Glaskapillarabschnitt und/oder einen Glassinterkörper und/oder Glaspulver und/oder Glaspaste umfassen. Die guten Absorptionseigenschaften im Vergleich zum zweiten Material des Spritzenkörpers 10 können dabei durch die Verwendung eines chemisch oder eines strukturell unterschiedlichen Materials erreicht werden. Insbesondere bevorzugt umfasst der Verbindungskörper 30 dotiertes Glas, beispielsweise mit Chrom-, Eisen- oder Nickeloxid dotiertes Glas und/oder einen Sinterkörper aus dotiertem Glas, insbesondere aus einem mit Chrom-, Eisen- oder Nickeloxid dotierten Glas und/oder einen Sinterkörper aus Glas, dessen Ausgangspulver beispielsweise mit färbenden Pigmenten gemischte wurde.

Bevorzugte Quellen für die Erzeugung von elektromagnetischer Strahlung für die selektive Erhitzung des Verbindungskörpers 30 umfassen jeweils eine oder mehrere UV-Strahlungsquellen, beispielsweise Quecksilberdampflampen und/oder Strahlungsquellen, die im sichtbaren Bereich abstrahlen, beispielsweise Xenon-Kurzbogen-Hochdrucklampen und/oder Infrarot-Strahlungsquellen, insbesondere Infrarot-Strahlungsquellen, die kurzwellige Infrarotstrahlung abgeben, beispielsweise Nd:YAG-Laser oder Wolfram-IR-Strahler und/oder Mikrowellenstrahlungsquellen, beispielsweise Magnetrons.

Gemäß einer besonders bevorzugten Ausführungsform der Erfindung kann neben der selektiven Erhitzung des Verbindungskörpers 30 jedoch auch der Spritzenkörper 10 durch die gleiche Strahlungsquelle 40 erhitzt werden. Dies ist insofern vorteilhaft, als dass es Spannungen, die in der Düse 11 durch die selektive Erhitzung des Verbindungskörpers 30 auftreten können, abmildern oder sogar weitgehend verhindert. Dabei ist beispielsweise der Unterschied in der Absorption von Spritzenkörper und Verbindungskörper so gewählt, dass der Verbindungskörper die Verbindungstemperatur und der Spritzenkörper Temperaturen erreicht, die nicht überhöhte Restspannungen gewährleisten, beispielesweise den unteren Kühlpunkt, aber unterhalb der Erweichungstemperatur bleibt.

Gemäß einer weiteren bevorzugten Ausführungsform der Erfindung kann neben der selektiven Erhitzung des Verbindungskörpers 30 jedoch auch der Spritzenkörper 10 durch eine Zusatzbeheizung 45 erhitzt werden. Dies kann insofern vorteilhaft sein, als es Spannungen, die in der Düse 11 durch die selektive Erhitzung des Verbindungskörpers 30 auftreten können, abmildern oder sogar weitgehend verhindern kann.

Bei dieser Zusatzheizung 45 kann es sich um eine konventionelle Heiztechnologie handeln. Vorzugsweise kann die Zusatzheizung durch Infrarot-Bestrahlung 46 oder einen Heißluftstrom 46 vorgenommen werden.

Gemäß einer bevorzugten Ausführungsform der Erfindung kann zur Herstellung eines Spritzenkörper-Kanülen-Verbunds wie in den Fig. 1 bis 8 dargestellt beispielsweise wie folgt vorgegangen werden.

Es wird ein Spritzenkörper 10 aus Glas mit dem Markennamen Fiolax® klar mit einer Gesamtlänge von 45 mm und einem Außendurchmesser von 8 mm bereitgestellt. Fiolax® klar weist eine Zusammensetzung in Gew.-% von 75% SiO₂, 10,5% B₂O₃, 7% Na₂O₃, 5% Al₂O₃, 1, 5% CaO und < 1% BaO auf. Darüber hinaus wird eine Kanüle 20 aus Edelstahl mit der Werkstoff-Nr. 1.4301 gemäß EN 10088-2 von 23 mm Gesamtlänge, einem Außendurchmesser von 0,5 mm verwendet. Außerdem ist ein Sinterkörper 30 aus Fiolax® klar, dotiert mit 5 Gew.-% Fe₂O₃ mit einer Höhe von 4 mm, einem Außendurchmesser von 1,45 mm und einem Innendurchmesser von 0,55 mm umfasst. Dieses System wird am Spritzenkörper 10 senkrecht auf einem Schlitten (nicht gezeigt) und die Kanüle 20 konzentrisch dazu senkrecht von oben in einer Nadelzange (nicht gezeigt) so fixiert, dass die freie Nadellänge zwischen Oberkante der Düse 11 und der Kanülenspitze 19 mm beträgt. Das System wird mit einer Geschwindigkeit von 1 cm/s bis 10 cm/s durch ein Durchlauf-Aggregat 40 gefahren, das kurzwellige IR-Strahlung 41 abgibt. In Höhe des Verbindungskörpers 30, hier des Sinterkörpers 30, wird innerhalb des kIR-Aggregats 40 von außen mit einem Wolfram-Halogen-IR-Strahler mit einer Farbtemperatur zwischen 1500 K und 3500 K auf die Düse 11 und den darin oder daran angeordneten Sinterkörper 30 eingestrahlt. Die Infrarot-Strahlungsleistung wird so eingestellt, dass der Sinterkörper 30 innerhalb von 1 bis 120 Sekunden, bevorzugt innerhalb von 1 bis 60 Sekunden und insbesondere bevorzugt innerhalb von 1 bis 45 Sekunden, ganz besonders bevorzugt innerhalb von 1 bis 30 Sekunden und absolut besonders bevorzugt 1 bis 10 Sekunden aufschmilzt und die Düse 11 mit der Kanüle 20 hermetisch dicht verbindet. Die kurze Aufschmelzzeit ermöglicht es, die Aufheizung lokal auf den Bereich des absorbierenden ersten Materials zu beschränken. So kann beispielsweise einer Schädigung der Kanüle entgegengewirkt werden.

Alternativ zu dem oben beschriebenen Beispiel mit Glas als Material des Verbindungskörpers und des Spritzenkörpers kann auch Kunststoff für zumindest eines dieser Elemente eingesetzt werden. Ein bevorzugter Kunststoff ist dabei COC. Die Temperaturen, die zur Erweichung des Kunststoffs erforderlich sind, sind typischerweise deutlich geringer als bei Gläsern.

Gemäß einer besonders bevorzugten Weiterbildung der Erfindung wird der gesamte Spritzenkörper 10 und gegebenenfalls auch die Kanüle 20 durch eine konventionelle Zusatzheizung 45 mit beispielsweise 500 W elektrischer Leistung oder eine Infrarotheizung oder eine andere geeignete Heizeinrichtung während der Infrarotbestrahlung des Verbundkörpers 30 auf mehrere hundert Grad Celsius erwärmt. Die Zusatzbeheizung des Spritzenkörpers 10 und ggf. der Kanüle 20 bewirkt, dass durch die lokale Infrarotbestrahlung 41 keine unzulässig hohen Spannungen im Spritzenkörper 10 oder in der Düse 11 entstehen.

Nach erfolgreicher Verschmelzung der Kanüle 20 mit dem Spritzenkörper 10 kann gegebenenfalls noch eine weitere thermische Nachbehandlung angeschlossen werden, die in den Figuren jedoch nicht abgebildet ist. Diese thermische Nachbehandlung dient dem Abbau von möglicherweise noch vorhandene Restspannungen im Spritzenkörper-Kanülen-Verbund 1. Im oben beschriebenen Ausführungsbeispiel weist der Spritzenkörper 10 einen durchschnittlichen thermischen Ausdehnungskoeffizienten α₍₂₀₋₃₀₀₎ von 4, 9 x 10⁻⁶/K, die Kanüle 20 aber einen durchschnittlichen thermischen Ausdehnungskoeffizienten von 10*10⁻⁶/K bis 20*10⁻⁶/K auf. Aus diesen Unterschieden können sich Spannungen entstehen, die mittels einer solchen thermischen Nachbehandlung zumindest deutlich gemildert und jedenfalls unterhalb des kritischen Niveaus für die Anwendung als Spritze gebracht werden.

Vorteilhaft kann auch der thermische Ausdehnungskoeffizient des ersten Materials des Verbindungskörpers zwischen dem des zweiten Materials der Spritzenkörpers 10 und dem des Materials der Kanüle 20 liegen. Das erste Material des Verbindungskörpers 30 wirkt dann vermittelnd auf den Verlauf der aufgebauten Spannung.

Anstelle der kurzwelligen IR-Strahlung 41 kann auch ein Nd:YAG-Laser 41 verwendet werden, um den Verbindungskörper 30 zumindest bereichsweise aufzuschmelzen. Die Anordnung von Spritzenkörper 10, Verbindungskörper 30 und Kanüle 20 entspricht dem oben Beschriebenen. Auch die verwendeten Materialien und Abmessungen sind identisch. Das angeordnete System aus Spritzenkörper 10, Kanüle 20 und Sinterkörper 30 wird mit einer Umdrehungsgeschwindigkeit von 1 bis 120 U/min, bevorzugt 1 bis 60 U/min und besonders bevorzugt mit 1 bis 30 U/min ganz besonders bevorzugt 1 bis 10 U/min rotiert. In Höhe des Sinterkörpers 30 wird radial von außen mit einem Laserstrahl einer Wellenlänge zwischen 800 nm und 1500 nm, bevorzugt zwischen 900 und 1100 nm auf den Sinterkörper 30 eingestrahlt. Dabei dient ein Scanner oder ein anderes geeignetes Gerät zur Strahlaufweitung, so dass eine Laserlinie von etwa 0,05 bis 12 mm Länge, oder beispielsweise einer Länge, die zumindest die Höhe des Verbindungskörpers 30 abdeckt, entsteht. Die Laserleistung wird so eingestellt, dass der Verbindungskörper 30, hier der Sinterkörper 30, innerhalb 1 s bis 60 s, bevorzugt innerhalb von 1 bis 30 s und besonders bevorzugt innerhalb von 1 bis 10 s aufschmilzt und die Düse 11 mit der Kanüle 20 hermetisch dicht verbindet. Eine konventionelle Zusatzheizung oder eine Infrarotheizung oder andere geeignete Heizeinrichtungen 45 erwärmt während der Laserbestrahlung 41 das gesamte System auf mehrere hundert Grad Celsius in jedem Fall soweit, dass durch die Laserbestrahlung 41 keine unzulässig hohen Spannungen im Spritzenkörper 10 oder in der Düse 11 entstehen. Es kann beispielsweise eine konventionelle Zusatzheizung 45 beispielsweise mit 500 W elektrischer Leistung verwendet werden.
Nach der erfolgten Verschmelzung zwischen Kanüle 20 und Spritzenkörper 10 kann eine thermische Nachbehandlung angeschlossen werden, um die restlichen Spannungen aus dem Spritzenkörper-Kanülen-Verbund 1 zu entfernen oder diese jedenfalls deutlich zu verringern.

Gemäß einer weiteren bevorzugten Ausführungsform der Erfindung kann die lokale Erhitzung des Verbindungskörpers 30 auch mittels einer Mikrowellen-Bestrahlung 41 erfolgen. Die Anordnung von Spritzenkörper 10, Verbindungskörper 30 und Kanüle 20 entspricht dem oben Beschriebenen. Auch die verwendeten Materialien, mit Ausnahme des Verbindungskörpers 30 sowie die Abmessungen der Bauteile sind identisch. Als Verbindungskörper 30 wird ein Sinterkörper aus Fiolax® klar verwendet, der mit 1 bis 95%, Eisenpulver gemischt ist. Das System aus Spritzenkörper 10, Kanüle 20 und Sinterkörper 30 wird in einem zylindrischen Mikrowellenresonator mit einem Innendurchmesser von beispielsweise 30 mm platziert und synchron mit einer Umdrehungsgeschwindigkeit zwischen 1 und 120 U/min rotiert. Über einen Mikrowellenhohlleiter 40 wird Mikrowellenstrahlung 41 mit einer Frequenz, die zwischen 0,8 GHz und 30 GHz, bevorzugt zwischen 0,85 und 3 GHz liegt, in den Mikrowellenresonator eingekoppelt.

Die Mikrowellenleistung kann durch Pulsen oder andere geeignete Regelungsmaßnahmen so eingestellt werden, dass der Sinterkörper 30 innerhalb einer Zeitspanne von 1 s bis 60 s, bevorzugt von 1 bis 30 s und insbesondere bevorzugt von 1 bis 10 s aufschmilzt und den Spritzenkörper 10 mit der Kanüle 20 hermetisch dicht verbindet.

Vorteilhaft erwärmt ein Heißluftstrom 46 mit einer Temperatur zwischen 30°C und 800°C, im vorliegenden Fall beispielsweise von 600°C, oder eine andere geeignete Heizeinrichtung 45 während der Mikrowellenbestrahlung 41 das gesamte System, umfassend zumindest den Spritzenkörper 10 und die Düse 11, auf mehrere hundert Grad Celsius und zumindest soweit, dass durch die Mikrowellenbestrahlung 41 keine unzulässig hohen Spannungen im Spritzenkörper 10 oder in der Düse 11 entstehen können. Dies kann beispielsweise erreicht werden, in dem das genannte System zumindest bis zu seinem unteren Kühlpunkt erwärmt wird. Nach erfolgter Verschmelzung der Kanüle 20 mit dem Spritzenkörper 10 oder der Düse 11 kann eine thermische Nachbehandlung wie oben beschrieben angeschlossen werden, um möglicherweise noch vorhandene Spannungen in der verbundenen Spritze zu relaxieren.

Vorteilhaft wird das System aus Spritzen- und Verbindungskörper vor der Mikrowellenbestrahlung auf eine Temperatur erwärmt, bei der die Mikrowellenstrahlung bevorzugt einkoppelt. Grundsätzlich sind verschiedene geometrische Anordnungen von Düse 11 und Verbindungskörper 30 möglich. In Fig. 2 ist eine Ausführungsform dargestellt, in der die Düse 11 eine distal orientierte Aussparung 12 aufweist, so dass ein röhrchenförmiger Verbindungskörper 30 vom freien Düsenende kommend, also aus distaler Richtung, eingebracht werden kann. Die Aussparung 12 ist dabei so dimensioniert, dass der Verbindungskörper 30 im eingebrachten Zustand im Wesentlichen mit dem distalen Rand der Düse 11 abschließt, jedoch nicht den gesamten Bereich der Düse 11 ausfüllt. Aussparungen 12 können vorteilhaft für die Stabilität der Spritzenkörper-Kanülen-Verbunds 1 sein, indem sie als Widerlager für den Verbindungskörper 30 dienen. Außerdem ermöglichen sie bei der Herstellung des Spritzenkörper-Kanülen-Verbunds 1 eine automatische Zentrierung beziehungsweise eine automatisch richtige Anordnung des Verbindungskörpers 30 in der Düse 11.

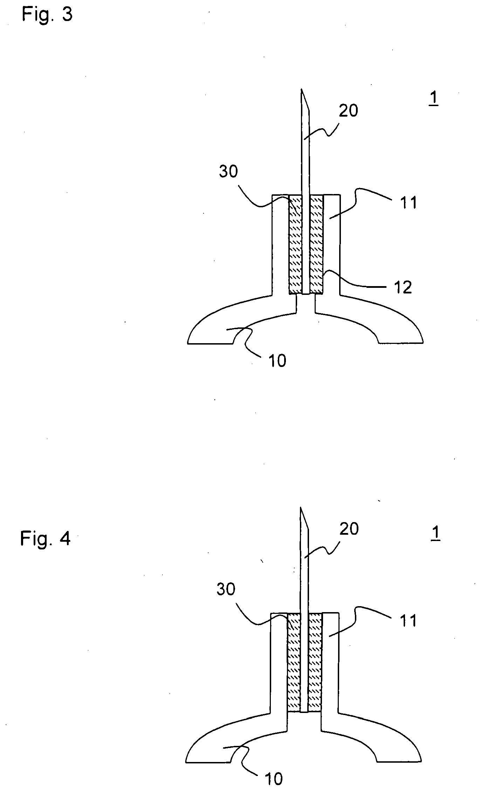

Figur 3 zeigt eine Anordnung des Verbindungskörpers 30 in der Düse 11 in einer distal orientierten Aussparung 12, wobei im Wesentlichen die gesamte Düse 11 durch den Verbindungskörper 30 ausgefüllt ist. Dies kann vorteilhaft sein, um sogen. Totraum zu vermeiden, also beispielsweise aufgrund der Spritzengeometrie nicht injizierbare Medikamentenvolumina.

In Fig. 4 ist dagegen eine Anordnung des Verbindungskörpers 30 in der Düse 11 dargestellt, die ohne Aussparung in der Düse auskommt.

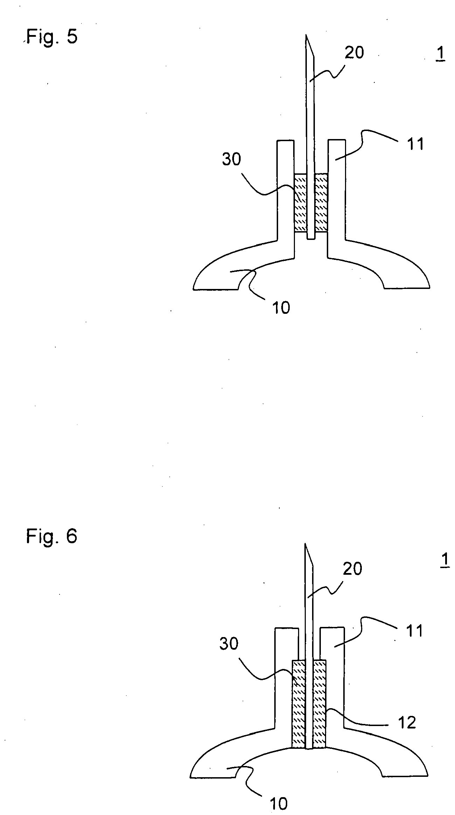

Figur 5 zeigt eine mittige Anordnung des Verbindungskörpers 30 in der Düse 11 ohne Aussparung.

Eine proximale Anordnung des Verbindungskörpers 30 in einer proximal orientierten Aussparung 12 in der Düse 11 ist in Fig. 6 dargestellt. Der Verbindungskörper 30 kann in diesem Fall vom Spritzenkörperinneren her in die Aussparung 12 in der Düse 11 eingebracht werden.

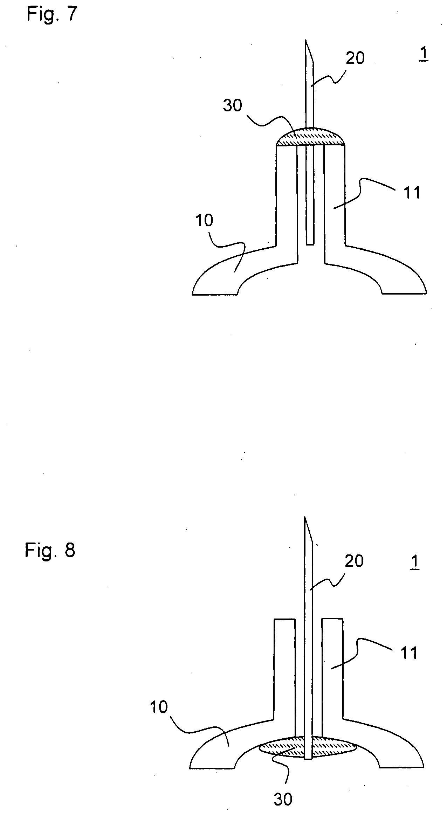

Die Ausführungsformen der Fig. 7 und 8 zeigen eine Anordnung, in der der Verbindungskörper 30 nicht in der Düse 11, sondern an der Düse 11 platziert ist. In Fig. 7 ist der Verbindungskörper 30 auf den distalen Düsenrand aufgesetzt. In Fig. 8 ist der Verbindungskörper 30 proximal auf die Düse 11 aufgesetzt und damit ins Spritzeninnere gerichtet.

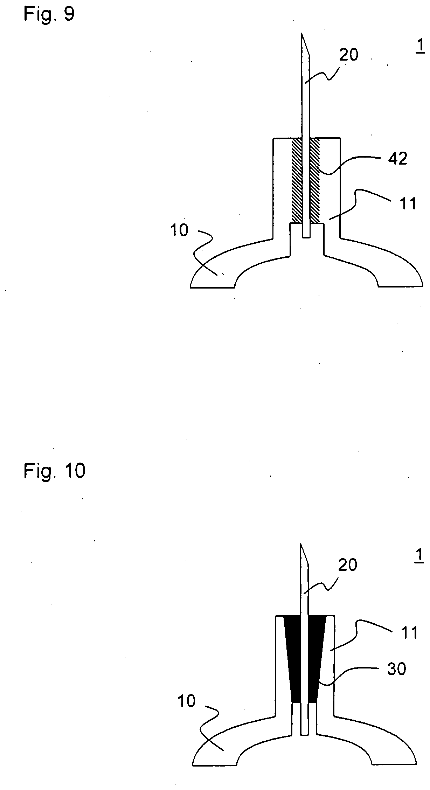

Figur 9 zeigt einen schematischen Querschnitt durch einen Spritzenkörper-Kanülen-Verbund 1, bei dem auf einen zusätzlichen Verbindungskörper 30 verzichtet wurde. Diese Ausführungsform kann durch chemische oder strukturelle Modifikation der Innenfläche der Düse 11 erzielt werden, so dass diese Bereiche 42 eine erhöhte Strahlungsabsorption gegenüber nicht modifizierten Spritzenteilen 41 aufweisen. Beispielsweise kann durch eine geeignete Oberflächenbehandlung, strahlungsaktive Ionen in die Oberfläche des Innenkanals der Düse diffundieren, was zur lokalen Erhöhung der Strahlungsabsorption im Bereich der eindiffundierten Ionen führt.

Figur 10 zeigt einen schematischen Querschnitt durch einen Spritzenkörper-Kanülen-Verbund 1. Der Verbindungskörper (30) ist hier konisch ausgeführt. Das hat den Vorteil, dass sich der Verbindungskörper (30) beim Assemblieren selbst zentriert und allseitiger Kontakt gewährleistet ist.

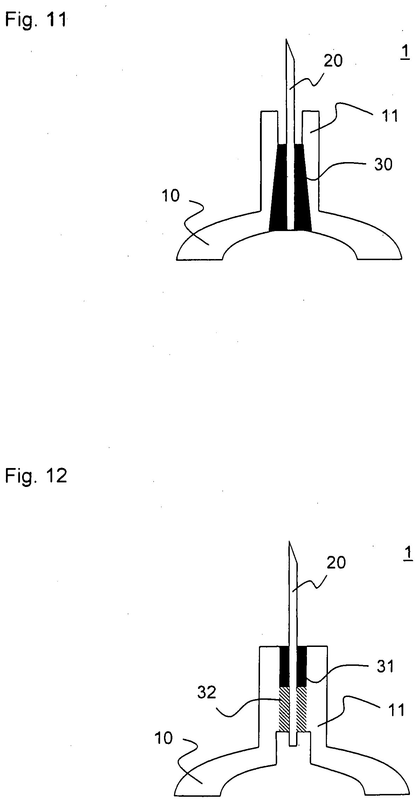

Figur 11 zeigt die gleiche Konfiguration wie Figur 10, nur ist hier der Verbindungskörper (30) anders orientiert. Der Verjüngung des Querschnitts verläuft hier in Richtung der Kanülenspitze.

Figur 12 zeigt einen schematischen Querschnitt durch einen Spritzenkörper-Kanülen-Verbund 1. Der Verbindungskörper 30 besteht hier aus zwei verschiedenen Gläsern 31, 32. Die Gläser können zur eindeutigen Markierung unterschiedlich gefärbt werden. Des Weiteren besteht die Möglichkeit, dass das Glas 31 eine niedrigere Schmelztemperatur hat als das Glas 32. Dies kann vorteilhaft genutzt werden, da Gläser mit niedriger Schmelztemperatur (z.B. zum Schutz der Nadel) oft den Nachteil, geringerer chemischer Stabilität aufweisen. Das Glas mit der höheren chemischen Stabilität schützt vor dem Kontakt mit dem Medikament.
Hat hingegen das Glas 31 eine höhere Schmelztemperatur als das Glas 32, bietet das den Vorteil, dass das Glas 32 nicht vollständig aufschmilzt und somit nicht in Kontakt mit der Nadel kommt. Dadurch wird die Nadel in diesem Bereich nicht so stark aufgeheizt und behält ihre Biegefestigkeit. Der Übergangsbereich zum aufgeschmolzenen Glas 32 wird mechanisch durch Verbindungskörper 31 geschützt. Die Biegefestigkeit der Nadel wird dadurch insgesamt erhöht.

## Patentansprüche

1. Verfahren zur Herstellung eines Spritzenkörper-Kanülen-Verbunds (1), dessen Inneres, das mit den aufzunehmenden Stoffen oder Medikamenten in Berührung kommt, ausschließlich anorganische Materialien aufweist, umfassend die folgenden Schritte:
- Auswählen eines Frequenzbereichs einer elektromagnetischen Strahlung (41) sowie eines ersten und eines zweiten Materials, so dass beim Bestrahlen des ersten und zweiten Materials (i) das erste Material mehr elektromagnetische Strahlung (41) als das zweite Material absorbiert und (ii) das erste Material aufschmilzt;
- Bereitstellen einer Kanüle (20) und eines Spritzenkörpers (10) mit Düse (11), wobei das erste Material im Bereich der Düse (11) bereitgestellt wird, zumindest einen Verbindungskörper (30) umfasst, und wobei der übrige Spritzenkörper (10) aus dem zweiten Material ausgebildet ist;
- Anordnen zumindest des Spritzenkörpers (10) und der Kanüle (20) derart, dass die Kanüle (20) mit dem Spritzenkörper (10) verbunden werden kann und im verbundenen Zustand eine hermetisch dichte Verbindung zwischen dem Spritzenkörper (10) und der Kanüle (20) vorliegt;
- zumindest lokales Bestrahlen des ersten Materials im Bereich der Düse (11) mit der elektromagnetischen Strahlung (41) des ausgewählten Frequenzbereichs zum zumindest bereichsweise Aufschmelzen des ersten Materials, wodurch die Verbindung zwischen Spritzenkörper (10) und Kanüle (20) hergestellt und die Kanüle (20) hermetisch dichtend im Spritzenkörper (10) fixiert wird;
- Abkühlen des Spritzenkörper-Kanülen-Verbunds (1).

2. Verfahren nach Anspruch 1, **dadurch gekennzeichnet, dass** als Material für den Spritzenkörper (10) und/oder den zumindest einen Verbindungskörper (30) Glas oder Kunststoff verwendet wird.

3. Verfahren nach einem der vorstehenden Ansprüche, **dadurch gekennzeichnet, dass** der zumindest eine Verbindungskörper (30) einen Glaskapillarabschnitt und/oder einen Sinterkörper aus Glas und/oder Glaspulver und/oder Glaspaste umfasst.

4. Verfahren nach einem der vorstehenden Ansprüche, **dadurch gekennzeichnet, dass** sich das erste Material des Verbindungskörpers (30) chemisch und/oder strukturell von dem zweiten Material des Spritzenkörpers (10) unterscheidet.

5. Verfahren nach einem der vorstehenden Ansprüche, **dadurch gekennzeichnet, dass** der zumindest eine Verbindungskörper (30) dotiertes Glas umfasst.

6. Verfahren nach einem der vorstehenden Ansprüche, **dadurch gekennzeichnet, dass** die elektromagnetische Strahlung (41) mittels einer oder mehreren UV-Strahlungsquellen und/oder Strahlungsquellen, die im sichtbaren Bereich abstrahlen und/oder Infrarot-Strahlungsquellen, insbesondere Infrarot-Strahlungsquellen, die kurzwellige Infrarotstrahlung abgeben, und/oder Mikrowellenstrahlungsquellen eingestrahlt wird.

7. Verfahren nach dem vorstehenden Anspruch, **dadurch gekennzeichnet, dass** als UV-Strahlungsquelle Quecksilberdampflampen, als Strahlungsquelle, die im sichtbaren Bereich abstrahlt, Xenon-Kurzbogen-Hochdrucklampen, als Infrarot-Strahlungsquelle Laser oder Temperaturstrahler oder als Mikrowellen-Strahlungsquelle ein Magnetrons verwendet werden.

8. Verfahren nach einem der vorstehenden Ansprüche, **dadurch gekennzeichnet, dass** beim Schritt des Herstellens der Verbindung zwischen Spritzenkörper (10) und Kanüle (20) der zumindest eine Verbindungskörper (30) zumindest teilweise aufgeschmolzen wird, wobei das aufgeschmolzene erste Material des Verbindungskörpers (30) nach dem Abkühlen an die Wandung des Spritzenkörpers (10) und/oder der Düse (11) des Spritzenkörpers (10) und um die Kanüle (20) anliegt, so dass die Kanüle (20) hermetisch dicht in den Spritzenkörper (10) eingefügt ist.

9. Verfahren nach einem der vorstehenden Ansprüche, **dadurch gekennzeichnet, dass** der Schritt des Herstellens der Verbindung zwischen Spritzenkörper (10) und Kanüle (20) das Erwärmen zumindest des Spritzenkörpers (10) umfasst, insbesondere wird der Spritzenkörper (10) erwärmt bis zumindest sein unterer Kühlpunkt bei einer Viskosität von η = 10^{14,5} d·Pas erreicht ist.

10. Verfahren nach einem der vorstehenden Ansprüche, **dadurch gekennzeichnet, dass** sich an den Schritt des Herstellens der Verbindung zwischen Spritzenkörper (10) und Kanüle (20) noch eine thermische Nachbehandlung anschließt, die noch vorhandene, durch unterschiedliche thermische Ausdehnungskoeffizienten der Materialien und/oder unterschiedlich stark erwärmte Bereiche des Spritzenkörpers (10) und der Kanüle (20) induzierte Restspannungen in zumindest dem Spritzenkörper (10) abbaut.

11. Spritzenkörper mit Kanüle, dessen Inneres, das mit den aufzunehmenden Stoffen oder Medikamenten in Berührung kommt, ausschließlich anorganische Materialien aufweist,
- umfassend einen Spritzenkörper (10) mit einer Düse (11), die eine Öffnung und einen Düsenkanal aufweist, und eine Kanüle (20),
- wobei die Kanüle (20) im Spritzenkörper (10) oder in der Düse (11) des Spritzenkörpers (10) fixiert ist und die Verbindung der Öffnung in der Düse (11) durch die die Kanüle (20) in das Innere des Spritzenkörpers (10) eingebracht ist, zur Kanüle (20) hermetisch abgedichtet ist, und
- wobei im Bereich der Düse (11) ein erstes Material vorhanden ist, das zumindest einen Verbindungskörper (30) aus einem der folgenden Materialien umfasst: ein Glaskapillarabschnitt, ein Glassinterkörper, Glaspulver oder Glaspaste, dotiertes Glas, so dass das erste Material während des Verbindungsprozesses zumindest teilweise mehr elektromagnetische Strahlung (41) absorbiert als ein zweites Material, aus dem der Spritzenkörper (10) gefertigt ist und die Innenseite des Düsenkanals sich schneller erwärmt als die Außenseite der Düse (11), und wobei
- die Kanüle (20) in das erste Material, welches mehr elektromagnetische Strahlung (41) absorbiert, eingeschmolzen ist.

12. Spritzenkörper mit Kanüle nach Anspruch 11, **dadurch gekennzeichnet, dass** der zumindest eine Verbindungskörper (30) in der Düse (11), insbesondere an deren oberem oder deren unterem Ende oder mittig, angeordnet ist.

13. Spritzenkörper mit Kanüle nach einem der Ansprüche 11 oder 12, **dadurch gekennzeichnet, dass** der zumindest eine Verbindungskörper (30) in eine zumindest einseitig offene Aussparung (12) in der Düse (11) eingepasst ist.

## Claims

1. Method for producing a syringe body-cannula assembly (1), of which the interior which comes into contact with the substances or medicaments to be taken up, comprises exclusively inorganic materials, comprising the steps of:
- selecting a frequency range of electromagnetic radiation (41) and a first material and a second material, so that, upon irradiation of the first and second material, (i) the first material absorbs more electromagnetic radiation (41) than the second material and (ii) the first material melts;
- providing a cannula (20) and a syringe body (10) having a nozzle (11), wherein the first material is provided in the region of the nozzle (11), comprises at least one connecting body (30), and wherein the remaining syringe body (10) is formed from the second material;
- arranging at least the syringe body (10) and the cannula (20) in such a manner that the cannula (20) can be connected to the syringe body (10) and in the connected state a hermetically sealed connection exists between the syringe body (10) and the cannula (20);
- at least locally irradiating the first material in the region of the nozzle (11) with the electromagnetic radiation (41) of the selected frequency range for melting the first material at least in regions, whereby the connection between the syringe body (10) and the cannula (20) is established and the cannula (20) is fixed in a hermetically sealing manner in the syringe body (10);
- cooling the syringe body-cannula assembly (1).

2. Method as claimed in claim 1, **characterised in that** glass or synthetic material is used as the material for the syringe body (10) and/or the at least one connecting body (30).

3. Method as claimed in any one of the preceding claims, **characterised in that** the at least one connecting body (30) comprises a glass capillary portion and/or a sintered body consisting of glass and/or glass powder and/or glass paste.

4. Method as claimed in any one of the preceding claims, **characterised in that** the first material of the connecting body (30) differs chemically and/or structurally from the second material of the syringe body (10).

5. Method as claimed in any one of the preceding claims, **characterised in that** the at least one connecting body (30) comprises doped glass.

6. Method as claimed in any one of the preceding claims, **characterised in that** the electromagnetic radiation (41) is irradiated by means of one or a plurality of UV radiation sources and/or radiation sources which emit in the visible range, and/or infrared radiation sources, in particular infrared radiation sources which output shortwave infrared radiation, and/or microwave radiation sources.

7. Method as claimed in the preceding claim, **characterised in that** mercury vapour lamps are used as the UV radiation source, xenon short arc high pressure lamps are used as the radiation source which emits in the visible range, lasers or thermal radiators are used as the infrared radiation source or a magnetron is used as the microwave radiation source.

8. Method as claimed in any one of the preceding claims, **characterised in that** in the step of establishing the connection between the syringe body (10) and the cannula (20) the at least one connecting body (30) is at least partially melted, wherein after cooling the molten first material of the connecting body (30) lies against the wall of the syringe body (10) and/or of the nozzle (11) of the syringe body (10) and around the cannula (20) so that the cannula (20) is inserted into the syringe body (10) in a hermetically sealed manner.

9. Method as claimed in any one of the preceding claims, **characterised in that** the step of establishing the connection between the syringe body (10) and the cannula (20) comprises heating at least the syringe body (10), in particular the syringe body (10) is heated until at least its lower cooling point is achieved at a viscosity of η = 10^{14.5} d · Pas.

10. Method as claimed in any one of the preceding claims, **characterised in that** the step of establishing the connection between the syringe body (10) and the cannula (20) is followed by a thermal after-treatment which reduces, in at least the syringe body (10), residual stresses which are still present and are induced by different thermal coefficients of expansion of the materials and/or regions of the syringe body (10) and the cannula (20) which are heated to different intensities.

11. Syringe body comprising a cannula, of which the interior which comes into contact with the substances or medicaments to be taken up, comprises exclusively inorganic materials,
- comprising a syringe body (10) having a nozzle (11) which has an opening and a nozzle channel, and a cannula (20),
- wherein the cannula (20) is fixed in the syringe body (10) or in the nozzle (11) of the syringe body (10), and the connection of the opening in the nozzle (11) is introduced through the cannula (20) into the interior of the syringe body (10), is hermetically sealed with respect to the cannula (20), and
- wherein present in the region of the nozzle (11) is a first material which comprises at least one connecting body (30) consisting of one of the following materials: a glass capillary portion, a sintered glass body, glass powder or glass paste, doped glass so that during the connection process the first material at least partially absorbs more electromagnetic radiation (41) than a second material, from which the syringe body (10) is manufactured and the inner side of the nozzle channel heats up more rapidly than the outer side of the nozzle (11), and wherein
- the cannula (20) is melted into the first material which absorbs more electromagnetic radiation (41).

12. Syringe body comprising a cannula as claimed in claim 11, **characterised in that** the at least one connecting body (30) is arranged in the nozzle (11), in particular at its upper or lower end or centrally.

13. Syringe body comprising a cannula as claimed in any one of claims 11 or 12, **characterised in that** the at least one connecting body (30) is fitted into an aperture (12), which is open at least on one side, in the nozzle (11).

## Revendications

1. Procédé de fabrication d'un ensemble corps de seringue-canule (1), dont l'intérieur, qui vient en contact avec les substances à accueillir ou les médicaments, comprend exclusivement des matériaux anorganiques, comportant les étapes suivantes :
- la sélection d'une plage de fréquences d'un rayonnement électromagnétique (41) ainsi que d'un premier et d'un deuxième matériau, de sorte que lorsque le premier et le deuxième matériau sont irradiés, (i) le premier matériau absorbe plus de rayonnement électromagnétique (41) que le deuxième matériau et (ii) le premier matériau fond ;
- la fourniture d'une canule (20) et d'un corps de seringue (10) comprenant une buse (11), où le premier matériau est mis à disposition dans la zone de la buse (11), comprend au moins un corps de liaison (30), et où le reste du corps de seringue (10) est fait du deuxième matériau ;
- l'agencement au moins du corps de seringue (10) et de la canule (20) de telle manière que la canule (20) puisse être reliée au corps de seringue (10) et qu'une fois ces derniers reliés, une liaison hermétiquement étanche soit présente entre le corps de seringue (10) et la canule (20) ;
- l'irradiation au moins locale du premier matériau dans la zone de la buse (11) au moyen du rayonnement électromagnétique (41) de la plage de fréquences sélectionnée pour faire fondre au moins en partie le premier matériau, de sorte que la liaison entre le corps de seringue (10) et la canule (20) est établie et que la canule (20) est fixée de manière hermétiquement étanche dans le corps de seringue (10) ;
- le refroidissement de l'ensemble corps de seringue-canule (1).

2. Procédé selon la revendication 1, **caractérisé en ce que** du verre ou une matière plastique est utilisé(e) comme matériau pour le corps de seringue (10) et/ou ledit au moins un corps de liaison (30).

3. Procédé selon l'une quelconque des revendications précédentes, **caractérisé en ce que** ledit au moins un corps de liaison (30) comprend une section de capillaire en verre et/ou un corps fritté en verre et/ou une poudre de verre et/ou une pâte de verre.

4. Procédé selon l'une quelconque des revendications précédentes, **caractérisé en ce que** le premier matériau du corps de liaison (30) se différencie chimiquement et/ou structurellement du deuxième matériau du corps de seringue (10).

5. Procédé selon l'une quelconque des revendications précédentes, **caractérisé en ce que** ledit au moins un corps de liaison (30) comprend du verre dopé.

6. Procédé selon l'une quelconque des revendications précédentes, **caractérisé en ce que** le rayonnement électromagnétique (41) est projeté au moyen d'une ou plusieurs sources de rayonnement UV et/ou sources de rayonnement qui émettent dans le spectre visible et/ou sources de rayonnement à infrarouge, en particulier des sources de rayonnement à infrarouge qui délivrent un rayonnement à infrarouge de faible longueur d'onde, et/ou sources de rayonnement à micro-ondes.

7. Procédé selon l'une quelconque des revendications précédentes, **caractérisé en ce que** des lampes à vapeur de mercure sont utilisées comme source de rayonnement UV, des lampes xénon à arc court et à haute pression sont utilisées comme source de rayonnement qui émet dans le spectre visible, un laser ou un radiateur thermique est utilisé comme source de rayonnement à infrarouge ou un magnétron est utilisé comme source de rayonnement à micro-ondes.

8. Procédé selon l'une quelconque des revendications précédentes, **caractérisé en ce que**, à l'étape de l'établissement de la liaison entre le corps de seringue (10) et la canule (20), ledit au moins un corps de liaison (30) est fondu au moins en partie, où le premier matériau fondu du corps de liaison (30), une fois refroidi, s'applique contre la paroi du corps de seringue (10) et/ou de la buse (11) du corps de seringue (10) et autour de la canule (20), de sorte que la canule (20) est insérée de manière hermétiquement étanche dans le corps de seringue (10).

9. Procédé selon l'une quelconque des revendications précédentes, **caractérisé en ce que** l'étape de l'établissement de la liaison entre le corps de seringue (10) et la canule (20) comprend le chauffage au moins du corps de seringue (10), en particulier le corps de seringue (10) est chauffé jusqu'à ce qu'au moins son point de refroidissement inférieur, pour une viscosité de η = 10^{14,5} d·Pas, ait été atteint.

10. Procédé selon l'une quelconque des revendications précédentes, **caractérisé en ce que**, à l'étape de l'établissement de la liaison entre le corps de seringue (10) et la canule (20), s'ajoute encore un post-traitement thermique, qui élimine dans au moins le corps de seringue (10) les tensions résiduelles encore présentes induites par les différents coefficients de dilatation thermiques des matériaux et/ou des zones chauffées plus ou moins intensément du corps de seringue (10) et de la canule (20).

11. Corps de seringue comprenant une canule, dont l'intérieur, qui vient en contact avec des substances à accueillir ou des médicaments, comprend exclusivement des matériaux anorganiques,
- comprenant un corps de seringue (10) comportant une buse (11), qui présente une ouverture et un canal de buse, et une canule (20),
- dans lequel la canule (20) est fixée dans le corps de seringue (10) ou dans la buse (11) du corps de seringue (10), et la liaison de l'ouverture dans la buse (11), par l'intermédiaire de laquelle la canule (20) est introduite à l'intérieur du corps de seringue (10), est étanchéifiée de manière hermétique par rapport à la canule (20), et
- dans lequel un premier matériau est présent dans la zone de la buse (11), qui comprend au moins un corps de liaison (30) fait d'un des matériaux suivants : une section de capillaire en verre, un corps fritté en verre, une poudre de verre ou une pâte de verre, un verre dopé, de sorte que le premier matériau, pendant le processus de liaison, absorbe au moins en partie plus de rayonnement électromagnétique (41) qu'un deuxième matériau dont est fait le corps de seringue (10), et la face intérieure du canal de buse chauffe plus rapidement que la face extérieure de la buse (11), et où
- la canule (20) est insérée par fusion dans le premier matériau, lequel absorbe plus de rayonnement électromagnétique (41).

12. Corps de seringue comprenant une canule selon la revendication 11, **caractérisé en ce que** ledit au moins un corps de liaison (30) est agencé dans la buse (11), en particulier à l'extrémité supérieure ou à l'extrémité inférieure ou de manière centrale.

13. Corps de seringue comprenant une canule selon l'une quelconque des revendications 11 et 12, **caractérisé en ce que** ledit au moins un corps de liaison (30) est emboîté dans la buse (11) dans un évidement (12) ouvert au moins par un côté.
